# EUROPÄISCHE PATENTSCHRIFT

(11) **EP 0 814 848 B1**
(45) Veröffentlichungstag und Bekanntmachung des Hinweises auf die Patenterteilung: **18.12.2002**
(21) Anmeldenummer: 96907479.8
(22) Anmeldetag: 14.03.1996
(51) Int. Cl.: A61K 49/00, A61P 43/00

(54) **MIKROPARTIKEL, DIESE ENTHALTENDE MITTEL FÜR DIE ULTRASCHALLDIAGNOSTIK SOWIE VERFAHREN ZUR HERSTELLUNG DER PARTIKEL UND MITTEL**
MICROPARTICLES, AGENTS CONTAINING SAID MICROPARTICLES FOR USE IN ULTRASOUND DIAGNOSTICS, AND PROCESSES FOR PRODUCING SAID PARTICLES AND AGENTS
MICROPARTICULES, PRODUITS LES CONTENANT UTILISES POUR LA TECHNIQUE DE DIAGNOSTIC A ULTRASONS, ET PROCEDE DE FABRICATION DE CES PARTICULES ET DE CES PRODUITS

(30) Priorität: 14.03.1995 DE 19510690
(43) Veröffentlichungstag der Anmeldung: 07.01.1998
(73) Patentinhaber: ACTIPAC Biosystems GmbH, 82152 Martinsried (DE)
(72) Erfinder: RÖSSLING, Georg, D-13465 Berlin (DE); ALBAYRAK, Celàl, D-10999 Berlin (DE); ROTHE, Matthias, D-12099 Berlin (DE)
(74) Vertreter: VOSSIUS & PARTNER
(86) Internationale Anmeldenummer: EP9601108
(87) Internationale Veröffentlichungsnummer: WO96028192

(56) Entgegenhaltungen:
- EP-A- 0 327 490
- WO-A-93/25241
- WO-A-93/25242

## Beschreibung

Die Erfindung betrifft den in den Patentansprüchen gekennzeichneten Gegenstand, daß heißt gasenthaltende Mikropartikel für die Ultraschalldiagnostik, deren Wandmaterial aus Blockcopolymeren von Polyestern von α-, β- oder γ-Hydroxycarbonsäuren mit linearen oder sternförmigen Polyethylenglykolen und gegebenenfalls Flüssigkristallen oder aus Polyestern von α-, β- oder γ-Hydroxycarbonsäuren und Flüssigkristallen aufgebaut ist, diese Partikel enthaltene Mittel für die Ultraschalldiagnostik, sowie Verfahren zur Herstellung der Mittel und Partikel.

Die Ultraschalldiagnostik hat in der Medizin wegen der komplikationslosen einfachen Handhabung sehr breite Anwendung gefunden. Ultraschallwellen werden an den Grenzflächen von unterschiedlichen Gewebearten reflektiert. Die dabei entstehenden Echosignale werden elektronisch verstärkt und sichtbar gemacht.

Die Darstellung von Blutgefäßen und innerer Organen mittels Ultraschall erlaubt im allgemeinen nicht die Darstellung des darin vorhandenen Blutflusses. Flüssigkeiten, insbesondere Blut, liefern nur dann Ultraschallkontrast, wenn Dichte- und Kompresibilitätsunterschiede zur Umgebung bestehen. Als Kontrastmittel werden in der medizinischen Ultraschalldiagnostik z. B. Gase enthaltende oder Gas produzierende Substanzen verwendet, da der Impedanzunterschied zwischen Gas und umgebendem Blut wesentlich größer ist, als der von Flüssigkeiten oder Festkörpern und Blut [Levine R.A., J. Am. Coll. Cardiol. 3 (1989) 28 ; Machi I.J. CU 11 (1983) 3].

Roelandt et al. [Ultrasound Med. Biol. 8 (1982) 471-492] beschreiben, daß durch periphere Injektionen von Lösungen, die feine Gasblasen enthalten, cardiale Echokontraste erzielt werden können. Diese Gasblasen werden in physiologisch verträglichen Lösungen z.B. durch Schütteln, andere Agitation oder durch Zusatz von Kohlendioxid erhalten. Sie sind jedoch hinsichtlich Anzahl und Größe nicht standardisiert und können nur unzulänglich reproduziert werden. Auch sind sie in der Regel nicht stabilisiert, so daß ihre Lebensdauer gering ist. Ihre mittleren Durchmesser liegen meist über Erythrocytengröße, so daß keine Lungenkapillarpassage mit nachfolgender Kontrastierung von Organen wie linkes Herz, Leber, Niere oder Milz möglich ist. Darüber hinaus eignen sie sich nicht für Quantifizierung, da sich das von ihnen erzeugte Ultraschallecho aus mehreren, nicht voneinander zu trennenden Prozessen wie Blasenentstehung, Koaleszenz und Auflösung zusammensetzt. So ist es z.B. nicht möglich, mit Hilfe dieser Ultraschall-Kontrastmittel über die Messung des Kontrastverlaufs im Myokard, Aussagen über die Transitzeiten zu gewinnen. Hierzu sind Kontrastmittel notwendig, deren Streukörper eine ausreichende Stabilität aufweisen.

In der EP 0 131 540 ist die Stabilisierung der Gasblasen durch Zucker beschrieben. Damit wird zwar die Reproduzierbarkeit und Homogenität des Kontrasteffektes verbessert, eine Lungenpassage überstehen diese Blasen jedoch nicht.

In den EP 0 122 624 und 0 123 235 wird beschrieben, daß der gasblasenstabilisierende Effekt von Zuckern, Zuckeralkoholen und Salzen durch Zusatz von grenzflächenaktiven Substanzen verbessert wird. Eine Lungenkapillargängigkeit und die Möglichkeit zur Darstellung des arteriellen Gefäßschenkels und verschiedener Organe wie Leber oder Milz ist bei diesen Ultraschallkontrastmitteln gegeben. Der Kontrasteffekt ist hierbei jedoch auf das Gefäßlumen beschränkt, da die Bläschen nicht von den Gewebezellen aufgenommen werden.

Keines der beschriebenen Ultraschall-Kontrastmittel verbleibt längere Zeit unverändert im Körper. Eine Organdarstellung mit ausreichender Signalintensität durch selektive Anreicherung nach i.v. Gabe oder Quantifizierung ist mit diesen Mitteln nicht möglich.

Eine Verkapselung von Gasen, wie beispielsweise Luft als Ultraschall-Kontrastmittel wird in der EP 0 224 934 beschrieben. Das hierbei verwendete Wandmaterial besteht aus Protein, insbesondere menschliches Serumalbumin mit den bekannten allergenen Eigenschaften, zu denen durch eine Denatuierung cytoxische Effekte hinzukommen können.

In der Patentschrift EP 0 327 490 werden gasenthaltende Mikropartikel für die Ultraschall-Diagnostik auf der Basis von biologisch abbaubaren, synthetischen Materialien beschrieben. Als bioabbaubare Polymere werden u.a. α-, β- oder γ-Hydroxycarbonsäuren offenbart. Die diagnostische Wirksamkeit daraus bereiteter Kontrastmittelpräparationen ist jedoch nicht in allen Fällen befriedigend.

Mir keinem der beschriebenen Ultraschall-Kontrastmittel ist eine Funktionsanalytik möglich.

Aufgabe der vorliegenden Erfindung war es daher, Verbindungen und daraus bereitete Mittel zu finden, die die Nachteile des Standes der Technik überwinden.

Diese Aufgabe wird durch die vorliegende Erfindung gelöst.

Es wurde gefunden, daß gashaltige Mikropartikel, deren Wandmaterial aus Blockcopolymeren von Polyestern von α-, β- oder γ-Hydroxycarbonsäuren mit linearen oder sternförmigen Polyethylenglykolen und gegebenenfalls Flüssigkristallen oder aus Polyestern von α-, β- oder γ-Hydroxycarbonsäuren und Flüssigkristallen aufgebaut ist, hervorragend als Ultraschallkontrastmittel geeignet sind.

### Als erfindungsgemäß einsetzbare Blockcopolymere seien beispielhaft genannt:

Glycolid-copolymere (PGA) mit Trimethylen Carbonat (TMC) oder Lactidcopolymere (PLA) mit Trimethylen Carbonat, Tetramethylenglycolid, δ-Valerolacton, ε-Caprolacton oder Blockcopolymere von Polyestern von Hydroxycarbonsäuren mit linearem- oder Star-Polyethylenglykol (PEG oder S-PEG), wie z.B. PLA/PEG AB-Blockcopolymere, PLA/PEG/PLA ABA-Blockcopolymere, S(3)-PEG-PLA Blockcopolymere, S(4)-PEG-PLA Blockcopolymere.

Als in dem polymeren Wandmaterial gegebenenfalls enthaltene flüssigkristalline Bestandteile seien beispielhaft genannt:
Polyoxyethylentrimethylolpropandistearat (PTDS), Polyoxyethylenglyceryldistearat (PGDS), Polyoxyethylendistearat (PDS), Polyoxyethylenstearyletherstearate (PSES), Polyoxyethylenlauryletherstearate (PLES), Monooxyethylentrimethylolpropantristearat (MTTS*) sowie Polyoxyethylenglyceryltristearat (PGTS*) [die genannten flüssigkristallinen Verbindungen sind von Nihon Emulsion Co Ltd. (Tokyo) zu beziehen].

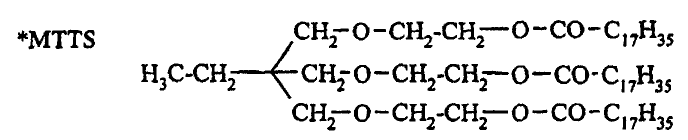

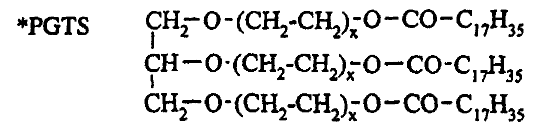

Erfindungsgemäße Mikropartikel, bei denen das Wandmaterial Flüssigkristalle enthält, können vorzugsweise in der Funktionsanalytik eingesetzt werden. So ändern die Flüssigkristalle in Abhängigkeit von der Temperatur ihren Aggregatzustand. Dieses führt bei entsprechender Temperatur zu einer Zerstörung der Partikelhülle, so daß das eingeschlossene Gas entweichen kann, was zu einem verschwinden des Ultraschallechos führt. Phasenübergangstemperaturen sind für eine Vielzahl kommerziell erhältlicher Flüssigkristalle bekannt und können den Herstellerangaben entnommen werden. Darüber hinaus kann die Verweilzeit der Mikropartikel im Blut gewünschtenfalls über die zugesetzte Menge an flüssigkristalliner Verbindung gesteuert werden. Der Anteil an Flüssigkristall in den erfindungsgemäßen Mikropartikeln beträgt 0-30% (Gewichtsprozent).

Als in den Partikeln enthaltene Gase kommen zur Anwendung Luft, Stickstoff, Edelgase, Distickstoffoxid, Kohlendioxid, Halogenkohlenwasserstoffe, gesättigte oder ungesättigte Kohlenwasserstoffe, Stickstoffdioxid und/oder Ammoniak.

Ein weiterer Aspekt der Erfindung betrifft ein Verfahren zur Herstellung von Mikropartikeln auf der Basis von Blockcopolymeren von Polyestern von α-, β- oder γ-Hydroxycarbonsäuren mit linearen oder sternförmigen Polyethylenglykolen und gegebenenfalls Flüssigkristallen sowie zur Herstellung von Mikropartikeln auf der Basis von α-, β- oder γ-Hydroxycarbonsäuren und Flüssigkristallen.

Derartige Partikel können erhalten werden, indem das jeweilige Polymer, gewünschtenfalls unter Zugabe der flüssigkristallinen Verbindung und gegebenenfalls eine oberflächenaktive Substanz in einem organischen Lösungsmitteln oder Lösungsmittelgemischen, von denen mindestens ein Lösungsmittel mit Wasser gut mischbar ist, gelöst wird, anschließend eine flüssige Perfluoro-Verbindung oder Wasser in dieser Lösung dispergiert wird und anschließend diese Dispersion in Wasser, das eine oberflächenaktive Substanz enthält, mittels eines Rührers dispergiert wird, wobei das Lösungsmittel durch Gaseinleitung und Anlegen eines Vakuums entfernt wird. Hierbei fallen Partikel aus, die zunächst noch Wasser bzw. die flüssige Perfluoro-Verbindung enthalten. Anschließend wird die Partikel enthaltende Suspension mit einem geeigneten pharmazeutisch akzeptablen Kyroprotektor vermischt und gefriergetrocknet, wobei die in den Partikeln befindliche Flüssigkeit weitgehend entweicht und nach Belüften des Lyophilisators durch das gewünschte Gas (in der Regel sterile Luft) ersetzt wird. Je nach Trocknungsdauer verbleibt gegebenenfalls eine geringe Menge der Flüssigkeit (Wasser bzw. Perfluoro-Verbindung) als Dampf in den Partikeln.

Als organische Lösungs- oder Lösungsmittelgemische für die Polymeren werden bevorzugt verwendet, Dichlormethan, Aceton, Ethylacetat, Methylacetat, Triacetin, Triethylcitrat, Ethyllactat, Isopropylacetat, Propylformiat, Butylformiat, Ethylformiat und/oder Methyllactat.

Als Perfluoro-Verbindung kommen vorzugsweise zur Anwendung Perfluoropentan, Perfluorohexan, Perfluoro-1,3-Dimethylcyclohexan, Perfluorocyclohexen, Perfluorodecalin oder Perfluoroether.

Als oberflächenaktive Substanz (Tensid) kommen zur Anwendung Substanzen aus der Gruppe der Poloxamere oder Poloxamine, Polyethylenglycol Alkylether, Polysorbate, Saccharoseester (Sisterna The Netherlands), Saccharoseester [Ryoto sugarester, (Tokyo)], Gelatine, Polyvinylpyrolidon, Fettalkoholpolyglycosid, Chaps (Serva), Chap (Calbiochem), Chapso (Calbiochem), Decyl-β-D-glycopyranosid, Decyl-β-Dmaltopyranosid, Dodecyl-β-D-maltopyranosid, Natriumoleat, Polyethylenglykol, Polyvinylalkohol oder deren Gemische.

Ein alternatives Verfahren besteht darin, daß (die) gewünschte(n) Polymer(e) und gegebenenfalls eine Aminosäure in mindestens einem organischen Lösungsmittel gelöst wird (werden), diese Lösung über eine Düse in eine Kolonne gesprüht wird, die mit einem überkritischen Gas befüllt ist oder von diesem durchströmt wird, wobei das Lösungsmittel vom überkritischen Gas aufgenommen wird.

Vorzugsweise wird bei dieser Verfahrensvariante zu dem jeweils verwendeten gelösten Polymer (Polyester) anstelle des Tensids eine Aminosäure gegeben. Als Aminosäuren kommen vorzugsweise zum Einsatz L-Lysin, L-Phenylalanin, L-Tryptophan sowie D,L-Phenylalanin.

Die Zugabe einer Perfluorverbindung ist in diesem Falle nicht erforderlich.

Als Lösungsmittel bzw. Lösungsmittelgemische eignen sich die vorgenannten Lösungsmittel.

Als überkritische Gase werden verwendet, Distickstoffoxid, Kohlendioxid, Halogenkohlenwasserstoffe, gesättigte oder ungesättigte Kohlenwasserstoffe, Stickstoffdioxid und/oder Ammoniak, wobei Kohlendioxid bevorzugt ist. Die überkritischen Gase können gegebenenfalls bis zu 10% Zusätze, wie z.B. niedere Alkohole wie z.B. Ethanol, Ester oder Gase wie z.B. Stickstoff enthalten.

Die Größe der resultierenden Partikel kann über Art, Größe und Form der Einspritzdüse, Arbeitsdruck und Temperatur in der Kolonne gesteuert werden. Eine Partikelgröße wie sie für ein intravenös appliziertes Ultraschallkontrastmittel erforderlich ist (< 10 µm) kann unter Verwendung einer Düse mit einem Düsendurchmesser von 0,5 mm und einem Sprühwinkel von 10° bei einem Arbeitsdruck zwischen 90 und 100 bar, vorzugsweise 94-96 bar und einer Temperatur von 36° C realisiert werden.

Ein weiterer Aspekt der Erfindung betrifft Kontrastmittel für die Ultraschalldiagnostik enthaltend die erfindungsgemäßen gashaltigen Mikropartikel.

Diese Mittel können hergestellt werden, indem die getrockneten Mikropartikel in einem pharmazeutisch akzeptablen Suspensionsmedium resuspendiert werden.

Als pharmazeutisch akzeptable Suspensionsmedien kommen infrage Wasser p.i., wäßrige Lösungen eines oder mehrere anorganische Salze wie physiologische Elektrolyt-Lösungen und Pufferlösungen, wie z.B. Tyrode, wäßrige Lösungen von Mono- oder Disacchariden wie Glucose oder Lactose, Zuckeralkoholen wie Mannit, die gegebenenfalls zusätzlich noch eine oberflächenaktive Substanz, z.B. aus der Gruppe der Polysorbate oder Polysaccharide, Polyvinylpyrrolidon, Polyethylenglykol, Saccharose Mono- und Diester oder Substanzen aus der Gruppe der Poloxamere oder Poloxamine oder deren Gemische und/oder einem physiologisch verträglichen mehrwertigen Alkohol wie Glycerin. Bevorzugt ist jedoch in für Injektionszwecke geeignetes Wasser.

Um die Sicherheit der Applikation zu erhöhen, kann unmittelbar vor Injektion eine Filtration der Suspension durchgeführt werden.

Die erfmdungsgemäßen Mikropartikel auf der Basis von Blockcopolymeren von Polyestern von α-, β- oder γ-Hydroxycarbonsäuren mit linearen oder sternförmigen Polyethylenglykolen und gegebenenfalls Flüssigkristallen oder auf der Basis von Polyestern von α-, β- oder γ-Hydroxycarbonsäuren und Flüssigkristallen, sowie daraus bereitete Kontrastmittel erfüllen alle Anforderungen, die an ein modernes Ultraschallkontrastmittel gestellt werden. Die in den Mitteln enthaltenen Partikel zeichnen sich durch die folgenden Vorteile aus:
- Sie werden schnell in-vivo abgebaut,
- Abbauprodukte sind toxikologisch unbedenklich,
- sie zirkulieren ausreichend lange im Blutkreislauf,
- sie sind in allen Modi der Ultraschalldiagnostik, insbesondere auch bei Modi bei denen nichtlineare Effekte ausgenutzt werden anwendbar,
- sie sind gut verträglich,
- sie zeigen eine einheitliche, steuerbare Größenverteilung,
- sie sind leicht herstellbar,
- sie sind ausreichend stabil um auch eine Lungenpassage zu überstehen und eignen sich damit auch fiir die Kontrastierung des linken Herzens und
- sie werden vom retikuloendothelialen System aufgenommen und eignen sich somit auch fiir die Kontrastierung der Leber und Milz.

Die Partikel und daraus bereitete Kontrastmittelpräparationen sind darüber hinaus gut verträglich, ohne ein allergenes Potential zu besitzen und verklumpen nicht im wäßrigen Medium. Weiterhin eignen sich die erfindungsgemäßen Mikropartikel deren Partikelhülle eine flüssigkristalline Verbindung enthält, für die Funktionsanalytik.

Nachfolgend eine Zusammenstellung der in der Anmeldung verwendeten Abkürzungen. Es bedeuten:
- PLA: Polylactid
- PGA: Polyglycolid
- TMC: Trimethylencarbonat
- S: Stern
- S(x): x-fach verzweigter Stern, wobei bei einer 3-fach Verzweigung die Verzweigungsstelle ein Stickstoffatom, bei einer 4-fach Verzweigung ein Ammonium (N⁺)-ion oder ein Kohlenstoffatom ist
- AB bzw. ABA: bezeichnet die Abfolge der monomeren Bausteine

Die nachfolgenden Beispiele dienen der näheren Erläuterung des Erfindungsgegenstandes, ohne ihn auf diese beschränken zu wollen.

### Beispiel 1

3 g Diblock Copolymer von Polylactid und Polyethylengylkol (PLA 9600/PEG 2100) herstellbar wie von K. J. Zhu et al. [Journal of applied Polymer science Vol. 39 (1990) 1- 9 oder Journal of Polymer science (Part A: Polymer letters) Vol. 24 (1986) 331-337], D. Shiaw-Guang Hu et al. [Polymer Bulletin 30, (1993) 669-676] oder X.M. Deng et al. [Journal of Polymer Science (Part C: Polymer letters) Vol. 28 (1990) 411-416] beschrieben - werden in 40 ml Methylenchlorid/Aceton (Volumenanteil 50:50) gelöst. 10 ml Perfluoropentan werden mittels Ultraturrax (10.000 Upm) 2 Minuten lang in der Polymerlösung dispergiert. Die entstandene Emulsion (0/0) wird in 400 ml 1%ige Gelatine Lösung, die auf 0°C temperiert ist, mittels eines mechanischen Rührers (Dispermat-FT, VMA-Getzmann GmbH), (1000 Upm) 30 Minuten lang dispergiert. Die Emulsion (0/0/W) wird in einem 2 1 Dreihalskolben, versehen mit einem Rührer (300 Upm) überführt, 3 h lang bei 20°C durch N₂-Einleitung, 3 Stunden lang bei 25°C durch Vakuum das Lösungsmittel entfernt. Anschließend wird die Suspension mit einem Kryoprotektor vermischt und gefriergetrocknet.
Das mit Wasser resuspendierte Lyophilisat enthält ultraschallaktive Mikropartikel von 0,1 bis 4 µm Durchmesser. Die Bestimmung der Partikeldurchmesser erfolgte mit einem LS-130 der Firma Coulter Electronics GmbH.

### Beispiel 2

Es wird wie in Beispiel 1 verfahren, wobei 1% Gelatinelösung durch 0,2% Gelatinelösung und das Diblockcopolymer durch Triblockcopolymer von Polyactid und Polyethylenglykol (ABA) ersetzt wird. Dieses Blockpolymer kann erhalten werden wie bei H. R. Kricheldorf et al. [Makromol. Chem. 194 (1993) 463], Li Youxin et al. [Journal of Controlled Release, 27 (1993) 247-257] beschreiben.
Das mit dem Wasser resuspendierten Lyophilisat enthält ultraschallaktive Mikropartikel von 0,1 bis 4 µm Durchmesser.

### Beispiel 3

Es wird wie in Beispiel 1 verfahren, wobei 1% Gelatinelösung durch 0,1% Gelatinelösung und das Diblockcopolymer durch Star-Polyethylenglykol- Polylactid Copolymer (S(3) PEG-PLA) ersetzt wurde. Dieses Polymer ist erhältlich wie bei K.J.Zhu [Journal of polymer science (Part A: Polymer chemistry) Vol. 27 (1989) 2151-2159] beschrieben.

Das mit dem Wasser resuspendierte Lyophilisat enthält ultraschallaktive Mikropartikel von 0,1 bis 2 µm Durchmesser.

### Beispiel 4

Es wird wie in Beispiel 1 verfahren, wobei das Diblockcopolymer durch ein Triblockcopolymer PLA-S(3)-PEG-PLA ersetzt wird. Dieses Polymer ist herstellbar wie von K.J. Zhu [Journal of polymer science: Part A Polymer chemistry, Vol. 27 (1989) 2151-2159] beschrieben.
Das mit dem Wasser resuspendierten Lyophilisat enthält ultraschallaktive Mikropartikel von 0,1 bis 5 µm Durchmesser.

### Beispiel 5

Es wird wie in Beispiel 1 verfahren, wobei das Diblockcopolymer durch Star-Polyethylenglykol-Polylactid (S(4)-PEG-PLA) ersetzt wurde. Dieses Polymer ist herstellbar wie von K.J. Zhu [Journal of polymer science: Part A Polymer chemistry, Vol. 27 (1989) 2151-2159] beschrieben.
Das mit dem Wasser resuspendierten Lyophilisat enthält ultraschallaktive Mikropartikel von 0,1 bis 2 µm Durchmesser.

### Beispiel 6

Es wird wie in Beispiel 1 verfahren, wobei das Diblockcopolymer durch ein Triblockcopolymer PLA-S(4)-PEG-PLA ersetzt wird. Dieses Polymer ist herstellbar wie von K.J. Zhu [Journal of polymer science: Part A Polymer chemistry, Vol. 27 (1989) 2151-2159] beschrieben.
Das mit dem Wasser resuspendierten Lyophilisat enthält ultraschallaktive Mikropartikel von 0,1 bis 4 µm Durchmesser.

### Beispiel 7

Es wird wie in Beispiel 1 verfahren, wobei in der Methylenchlorid/Acetonlösung außer dem Diblockcopolymer auch 300 mg Flüssigkristall MTTS [Nihon Emulsion Co Ltd. (Tokyo)] gelöst werden.
Das mit dem Wasser resuspendierte Lyophilisat enthält ultraschallaktive Mikropartikel von 0,3 bis 7 µm Durchmesser.

### Beispiel 8

Es wird wie in Beispiel 1 verfahren, wobei in der Methylenchlorid/Acetonlösung außer dem Diblockcopolymer auch 300 mg Flüssigkristall PGTS [Nihon Emulsion Co Ltd. (Tokyo)] gelöst werden. Das mit dem Wasser resuspendierte Lyophilisat enthält ultraschallaktive Mikropartikel von 0,3 bis 7 µm Durchmesser.

### Beispiel 9

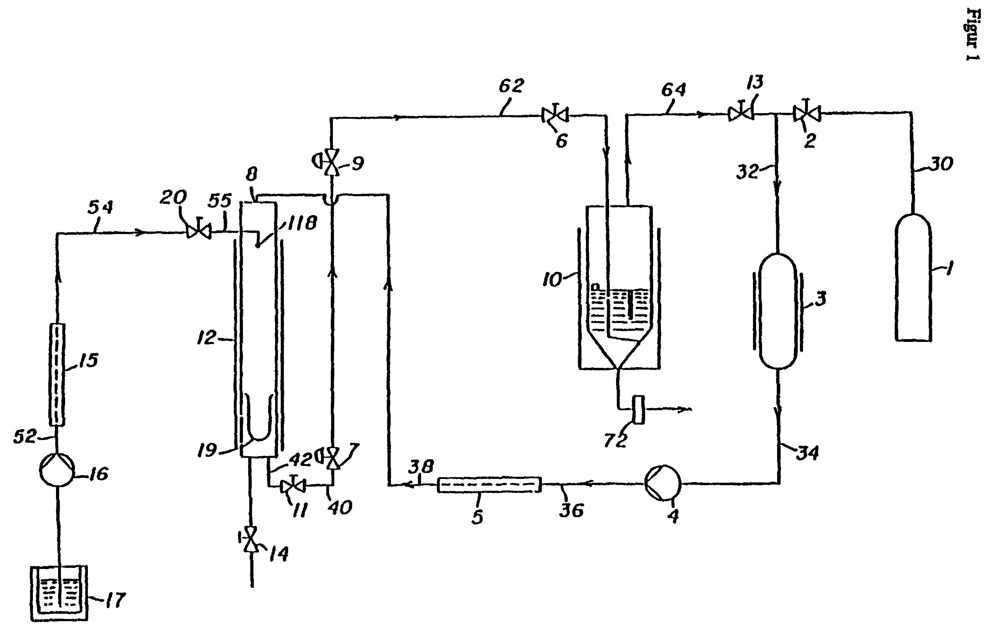

0,375 g L-Lysin (Aldrich) werden in 25 ml Eisessig (Merck) gelöst und mit 2,5 g PLA-PEG (Böhringer Ingelheim) gelöst in 75 ml Dichlormethan (Merck) versetzt. Die vereinigten Lösungen in einer Apparatur wie in Figur 1 abbgebildet weiter behandelt.

Dazu werden die vereinigten Lösungen zunächst in ein Reservoir (17) gegeben und die Anlage über Ventil (2) und Leitung (30) aus einer Vorratsflaschen mit dem Gas befüllt. Mittels einer Kolbenhubpumpe (16) wird die Lösung aus dem Reservoir (17) nach Durchströmen von Leitung (52), einem Wärmetauscher (15), einer Leitung (54), einem Ventil (20) und schließlich Leitung (55), der Düse (118) zugeleitet. Mit einem Druck von 94-96 bar wird die Copolymer enthaltende Lösung durch eine konventionelle Einstoffdüse (118) [Typ Schlick 121 V] in die Kolonne (12) versprüht, wobei gleichzeitig CO₂ im überkritischen Zustand mit 90 bar/36° C und einem Durchsatz von 8,9 kg/h im Gleichstrom, über Einlaß (8), durch die Kolonne geleitet wird. Die Düse hat einen Durchmesser von 0,5 mm, der Sprühwinkel beträgt 10°.

Entsprechend der hohen Affinität des überkritischen CO₂ zum Lösungsmittel wird den primär gebildeten Tröpfchen Lösungsmittel entzogen. Zurück bleiben kugelförmige feste Polymerpartikeln.

Die weiteren Schritte dienen im wesentlichen der Reinigung und Rückführung des Lösungsmittelbeladenen CO₂, haben aber mit der Herstellung der Partikel nichts mehr zu tun. Die Aufbereitung des CO₂ kann wie folgt geschehen. Das mit dem Lösemittel beladene Gas entströmt durch Leitungen (42) und (40), gesteuert durch 2 Magnetventile (7 und 9), dem Kolonnenende und wird auf 60 bar entspannt. Die Ventile sind so geschaltet, daß die pro Zeiteinheit in die Kolonne einströmende Menge des fluiden Gases unter Aufrechthaltung des Arbeitsdruckes der Kolonne entströmen kann. Das durch die Entspannung auf 60 bar abgekühlte, mit Lösungsmittel beladene CO₂ wird mittels Leitung (62) in den auf 21° C temperierten Separator (10) geleitet, wo sich das Lösungsmittelgemisch infolge der unter diesen Bedingungen stark verminderten Löslichkeiten im CO₂ abscheidet. Das vom Lösungsmittelgemisch befreite CO₂ wird mittels Leitung (64 und 32) durch Druck- und Temperaturerhöhung (3 und 4) erneut in den überkritischen Zustand überführt (90 bar, 36° C) und zur weiteren Trocknung der entstandenen Partikel erneut der Kolonne über Leitung (34), Flüssiggaspumpe (4), Leitung (36), Wärmetauscher (5), Leitung (38) durch Einlaß (8) zugeführt.

Die Entnahme des im Separator (10) abgetrennten Lösungsmittelgemischs erfolgt nach Abtrennung des Separators (10) aus dem Kreislauf durch Ventile (6) und (13) und Entspannung auf Atmosphärendruck über das Ventil (72).

Nachdem die gesamte im Reservoir enthaltene Menge an gelöstem Polymer versprüht wurde (Zeitdauer je nach Druck 20 bis 50 min), wird noch solange CO₂ durch die Kolonne geleitet, bis im Separator (10) keine Lösungsmittelreste mehr zurückgewonnen werden können.

Nach Abschluß des Trocknungsvorganges wird der CO₂-Strom zur Kolonne abgestellt, die Kolonne über die Ventile (11) und (14) auf Atmosphärendruck entspannt und die Partikel am unteren Kolonnenende (19) entnommen.

Es werden ultraschallaktive, gashaltige Mikropartikel mit einem Durchmesser von 1-10 µm erhalten.

### Beispiel 10-16

Die Herstellung weiterer Mikropartikel erfolgt analog zu Beispiel 9. Die jeweils verwendeten Polymeren sowie weitere Daten sind in Tabelle 1 zusammengefaßt.

In allen Fällen werden ultraschallaktive, gashaltige Mikropartikel erhalten.
Die erhaltenen Partikel sind steril und frei von Lösemittelrückständen, Polymerisationskatalysatoren oder Initiatormolekülen.
Sie besitzen eine gleichmäßige Teilchengrößenverteilung von 1 bis 10 µm.

Die Polymere werden jeweils in 75 ml Dichlormethan (Merck) und 25 ml Eisessig gelöst, die jeweilige Menge Aminosäure wird zugegeben. D,L-Phenylalanin ist geeigneterweise in bis 50 ml Ethanol (Merck) vorzulösen.

**Tabelle 1**

| Versuchsnummer | Polymer | Einwaage Polymer [g] | Aminosäure | Einwaage Aminosäure [g] |
|---|---|---|---|---|
| 10 | Poly-(D,L-Lactid-cotrimethylcarbonat) | 2,0 | L-Lysin | 0,3 |
| 11 | Poly-(D,L-lactidcoglycolid)-PEG-blockpolymer | 1,0 | L-Phenyl-alanin | 0,15 |
| 12 | Poly-PLA-PEG-PLAblockpolymer | 2,0 | D,L-Phenyl-alanin | 0,3 |
| 13 | S(3)-PEG-PLA-Sternblockcopolymer | 2,2 | L-Tryptophan | 0,35 |
| 14 | S(4)-PEG-PLA-Sternblockcopolymer | 3,0 | L-Lysin | 0,25 |
| 15 | PLA-S(4)-PEG-PLA-Sternblockcopolymer | 2,0 | L-Lysin | 0,2 |
| 16 | PLA-S(3)-PEG-PLA-Sternblockcopolymer | 2,0 | L-Lysin | 0,15 |

## Patentansprüche

1. Gashaltige Mikropartikel für die Verwendung in der Ultraschalldiagnostik, **dadurch gekennzeichnet, daß** das Wandmaterial der Partikel entweder aus Blockcopolymeren von Polyestern von α-, β- oder γ-Hydroxycarbonsäuren mit linearen oder stemförmigen Polyethylenglykolen, die gegebenenfalls flüssigkristalline Verbindungen enthalten, oder aus Polyestern von α-, β- oder γ-Hydroxycarbonsäuren und flüssigkristallinen Verbindungen aufgebaut ist.

2. Gashaltige Mikropartikel nach Anspruch 1, **dadurch gekennzeichnet, daß** den Blockcopolymeren von Polyestern von α-, β- oder γ-Hydroxycarbonsäuren mit linearen oder sternförmigen Polyethylenglykolen eine flüssigkristalline Verbindung zugesetzt ist.

3. Gashaltige Mikropartikel nach Anspruch 1 oder 2, **dadurch gekennzeichnet, daß** das Wandmaterial aufgebaut ist aus Glycolid-copolymeren mit Trimethylen Carbonat oder
Lactid-copolymeren mit Trimethylen Carbonat, Tetramethylenglycolid, δ-Valerolacton oder ε-Caprolacton oder
Blockcopolymeren von Polyestern von Hydroxycarbonsäuren mit linearen- oder Star-Polyethylenglykol, PLA/PEG AB-Blockcopolymeren, PLA/PEG/PLA ABA-Blockcopolymeren, S(3)-PEG-PLA Blockcopolymeren oder S(4)-PEG-PLA Blockcopolymeren.

4. Gashaltige Mikropartikel nach Anspruch 1, **dadurch gekennzeichnet, daß** als flüssigkristalline Verbindung Polyoxyethylentrimethylolpropandistearat, Polyoxyethylenglyceryldistearat, Polyoxyethylendistearat, Polyoxyethylenstearyletherstearate, Polyoxyethylenlauryletherstearate, Monooxyethylentrimethylolpropantristearat oder Polyoxyethylenglyceryltristearat enthalten ist.

5. Gashaltige Mikropartikel nach Anspruch 4, **dadurch gekennzeichnet, daß** der Anteil der flüssigkristallinen Verbindung 0-30 Gewichtsprozent beträgt.

6. Gashaltige Mikropartikel nach Anspruch 1 bis 5 enthaltend als Gas Luft, Stickstoff, Edelgase, Distickstoffoxid, Kohlendioxid, Halogenkohlenwasserstoffe, gesättigte oder ungesättigte Kohlenwasserstoffe, Stickstoffdioxid und/oder Ammoniak.

7. Verwendung von Mikropartikeln nach Anspruch 4 oder 5 Herstellung eines Mittels für die Funktionsanalytik.

8. Verfahren zur Herstellung von gasenthaltenden Mikropartikeln für die Ultraschalldiagnostik, deren Wandmaterial aus Blockcopolymeren von Polyestern von α-, β- oder γ-Hydroxycarbonsäuren mit linearen oder. sternförmigen Polyethylenglykolen und gegebenenfalls flüssigkristallinen Verbindungen oder aus Polyestern von α-, β- oder γ-Hydroxycarbonsäuren und flüssigkristallinen Verbindung aufgebaut ist, **dadurch gekennzeichnet, daß** das gewünschte Polymer, gewünschtenfalls die flüssigkristalline Verbindung und gegebenenfalls eine oberflächenaktive Substanz in einem organischen Lösungsmittel oder Lösungsmittelgemischen, von denen mindestens ein Lösungsmittel mit Wasser gut mischbar ist, gelöst wird, anschließend eine bei Körpertemperatur gasförmige Perfluoro-Verbindung oder Wasser in dieser Lösung dispergiert wird und anschließend diese Dispersion in Wasser, das eine oberflächenaktive Substanz enthält, mittels eines Rührers dispergiert wird, wobei das Lösungsmittel durch Gaseinleitung und Anlegen eines Vakuums entfernt wird und abschließend die so erhaltene Suspension mit einem geeigneten pharmazeutisch akzeptablen Kryoprotektor vermischt und gefriergetrocknet wird.

9. Verfahren nach Anspruch 8, **dadurch gekennzeichnet, daß** als oberflächenaktive Substanz Substanzen aus der Gruppe der Poloxamere oder Poloxamine, Polyethylenglycol Alkylether, Polysorbate, Saccharoseester, Gelatine, Polyvinylpyrolidon, Fettalkoholpolyglycosid, Chaps, Chap, Chapso , Decyl-β-D-glycopyranosid, Decyl-β-D-maltopyranosid, Dodecyl-β-D-maltopyranosid, Natriumoleat, Polyethylenglykol, Polyvinylalkohol oder deren Gemische verwendet werden.

10. Verfahren nach Anspruch 8 oder 9, **dadurch gekennzeichnet, daß** als perfluoro-Verbindung Perfluoropentan, Perfluorohexan, Perfluoro-1,3-Dimethylcyclohexan, Perfluorocyclohexen, Perfluorodecalin und/oder Perfluoroether verwendet wird.

11. Verfahren zur Herstellung von gasenthaltenden Mikropartikeln für die Ultraschalldiagnostik, deren Wandmaterial aus Blockcopolymeren von Polyestern von α-, β- oder γ-Hydroxycarbonsäuren mit linearen oder sternförmigen Polyethylenglykolen aufgebaut ist, **dadurch gekennzeichnet, daß** das (die) gewünschte(n) Polymer(e) und gegebenenfalls eine Aminosäure in mindestens einem organischen Lösungsmittel gelöst wird (werden), diese Lösung über eine Düse in eine Kolonne gesprüht wird, die mit einem überkritischen Gas befüllt ist oder von diesem durchströmt wird, wobei das Lösungsmittel vom überkritischen Gas aufgenommen wird.

12. Verfahren nach Anspruch 11, **dadurch gekennzeichnet, daß** als überkritisches Gas Distickstoffoxid, Kohlendioxid, Halogenkohlenwasserstoffe, gesättigte oder ungesättigte Kohlenwasserstoffe, Stickstoffdioxid und/oder Ammoniak verwendet wird.

13. Verfahren nach Anspruch 11 oder 12, **dadurch gekennzeichnet, daß** als Aminosäure L-Lysin, L-Phenylalanin, L-Tryptophan oder D,L-Phenylalanin verwendet wird.

14. Verfahren nach Anspruch 8 bis 13, **dadurch gekennzeichnet, daß** als organisches Lösungsmittel für das Polymer Dichlormethan, Aceton, Ethylacetat, Methylacetat, Triacetin, Triethylcitrat, Ethyllactat, Isopropylacetat, Propylformiat, Butylformiat, Ethylformiat und/oder Methyllactat verwendet wird.

15. Verfahren zur Herstellung von Kontrastmitteln für die Ultraschalldiagnostik, **dadurch gekennzeichnet, daß** Mikropartikel nach Anspruch 1-6 in einem pharmazeutisch verträglichen Suspensionsmedium suspendiert werden.

## Claims

1. Gas containing microparticles for use in ultrasonic diagnosis, **characterized in that** the wall material of the particles is built up from block copolymers of polyesters of α-, β- or γ-hydroxycarboxylic acids with linear or star-shaped polyethylene glycols which optionally contain liquid crystals or from polyesters of α-, β- or γ-hydroxycarboxylic acids and liquid crystal compositions.

2. Gas containing microparticles according to claim 1, **characterized in that** a liquid crystal compound is added to the block copolymers of polyesters of α-, β- or γ-hydroxycarboxylic acids with linear or star-shaped polyethylene glycols.

3. Gas containing microparticles according to claim 1 or 2, **characterized in that** the wall material is built up from glycolide-copolymers with trimethylene carbonate or lactide-copolymers with trimethylene carbonate, tetramethylene glycolide, δ-valerolactone or ε-caprolactone or block copolymers of polyesters of hydroxcarboxylic acids with linear- or star-polyethylene glycol, PLA/PEG AB-block copolymers, PLA/PEG/PLA ABA-block copolymers, S(3)-PEG-PLA block copolymers or S(4)-PEG-PLA block copolymers.

4. Gas containing microparticles according to claim 1, **characterized in that** the liquid-crystalline compound is selected from the group consisting of polyoxyethylene trimethylolpropanedistearate, polyoxyethylene glyceryl distearate, polyoxyethylene distearate, polyoxyethylene stearyl ether stearate, polyoxyethylene lauryl ether stearate, monooxyethylene trimethylolpropane tristearate or polyoxyethylene glyceryl tristearate.

5. Gas containing microparticles according to claim 4, **characterized in that** the proportion of liquid-crystalline compound amounts to 0 to 30 % by weight.

6. Gas containing microparticles according to claims 1 to 5 comprising a gas from the group consisting of air, nitrogen, noble gases, dinitrogen oxide, carbon dioxide, halogenated hydrocarbons, saturated or unsaturated hydrocarbons, nitrogen dioxide, and/or ammonia.

7. Use of microparticles according to claim 4 or 5 for the manufacture of a preparation for functional analysis.

8. Process for the production of gas containing microparticles for ultrasonic diagnosis, whose wall material is built up from block copolymers of polyesters of α-, β- or γ-hydroxycarboxylic acids with linear or star-shaped polyethylene glycols and optionally liquid-crystalline compounds or from polyesters of α-, β- or γ-hydroxycarboxylic acids and liquid-crystalline compounds, **characterized in that** the desired polymer, if desired, the liquid-crystalline compound, and optionally a surface-active substance are dissolved in an organic solvent or solvent mixtures, of which at least one solvent is readily water-miscible, then a perfluoro compound which is gaseous at body temperature or water is dispersed in this solution and then this dispersion is dispersed in water which contains a surface-active substance using a stirring mechanism, whereby the solvent is removed by pumping in gas and applying a vacuum, and finally the thus obtained suspension is mixed with a suitable pharmaceutically acceptable cryoprotector and freeze-dried.

9. Process according to claim 8, **characterized in that** the surface-active substance is selected from the group consisting of poloxamers or poloxamines, polyethylene glycol alkyl ethers, polysorbates, saccharose esters, gelatin, polyvinylpyrrolidone, fatty alcohol polyglycoside, Chaps, Chap, Chapso, decyl-β-D-glycopyranoside, decyl-β-D-maltopyranoside, dodecyl-β-D-maltopyranoside, sodium oleate, polyethylene glycol, polyvinyl alcohol or mixtures thereof.

10. Process according to claim 8 or 9, **characterized in that** the perfluoro compound is selected from the group consisting of perfluoropentane, perfluorohexane, perfluoro-1,3-dimethylcyclohexane, perfluorocyclohexene, perfluorodecalin and/or perfluoroether.

11. Process for the production of gas containing microparticles for ultrasonic diagnosis whose wall material is built up from block copolymers of α-, β- or γ-hydroxycarboxylic acids with linear- or star-shaped polyethylene glycols, **characterized in that** the desired polymer(s) and optionally an amino acid is (are) dissolved in at least one organic solvent, this solvent is sprayed via a nozzle into a column, which is filled or passed through with supercritical gas, wherein the solvent is taken up by the supercritical gas.

12. Process according to claim 11, **characterized in that** the supercritical gas is selected from the group consisting of dinitrogen oxide, carbon dioxide, halogenated hydrocarbons, saturated or unsaturated hydrocarbons, nitrogendioxide and/or ammonia.

13. Process according to claim 11 or 12, **characterized in that** the amino acid is selected from the group consisting of L-lysin, L-phenylalanin, L-tryptophane or D,L-phenylalanin.

14. Process according to claims 8 to 13, **characterized in that** the organic solvent is selected from the group consisting of dichlormethane, acetone, ethyl acetate, methyl acetate, triacetin, triethyl citrate, ethyl lactate, isopropyl acetate, propyl formiate, butyl formiate, ethyl formiate and/or methyl lactate.

15. Process for the production of contrast media for ultrasonic diagnosis, **characterized in that** the microparticles according to claims 1 to 6 are suspended in a pharmaceutically acceptable suspension medium.

## Revendications

1. Microparticules à teneur en gaz pour l'utilisation pour le diagnostic échographique, **caractérisée en ce que** le matériel de la paroi de la particule est constitué soit de copolymères à blocs de polyesters d'acides a-, β-, ou γ-hydroxycarboxyliques avec des polyéthylène glycols linéaires ou étoilés, qui le cas échéant contiennent des composés liquides cristallins ou est constitué de polyesters d'acides a-, β-, ou γ-hydroxycarboxyliques et de composés liquides cristallins.

2. Microparticules à teneur en gaz selon la revendication 1, **caractérisée en ce qu'**un composé liquide cristallin est ajouté aux copolymères à blocs de polyesters d'acides a-, β-, ou γ-hydroxycarboxyliques avec des polyéthylène glycols linéaires ou étoilés.

3. Microparticules à teneur en gaz selon la revendication 1 ou 2, **caractérisée en ce que** le matériau de la paroi est constitué de copolymère de glycolide avec le triméthylène carbonate ou
des copolymères de lactide avec le triméthylène carbonate, le tétraméthylène glycolide,
la δ-valérolactonc ou l'ε-caprolactone ou
des copolymères à blocs de polyesters d'acides hydroxycarboxyliques avec du polyéthylène glycol linéaire ou étoilé, des copolymères à blocs PLA/PEG AB, des copolymères à blocs PLA/PEG/PLA ABA, des copolymères à blocs S(3)-PEG-PLA ou des copolymères à blocs S(4)-PEG-PLA.

4. Microparticules à teneur en gaz selon la revendication 1, **caractérisée en ce qu'**elles contiennent comme composé liquide cristallin le distéarate de polyoxyéthylène triméthylolpropane, le distéarate de polyoxyéthylène glycéryle, le distéarate de polyxoxyéthylène, le stéarate de polyoxyéthylène stéaryléther, le stéarate de polyoxyéthytène lauryléther, le tristéarate de monooxyéthylène triméthylolpropane ou le tristéarate de polyoxyéthylène glycéryle.

5. Microparticules à teneur en gaz selon la revendication 4, **caractérisée en ce que** la proportion de composé liquide cristallin est comprise entre 0 et 30 pour cent en poids.

6. Microparticules à teneur en gaz selon les revendications 1 à 5, contenant comme gaz de l'air, de l'azote, un gaz rare, de l'oxyde azoté, du dioxyde de carbone, des hydrocarbures halogénés, des hydrocarbures saturés ou insaturés, du dioxyde d'azote et/ou du gaz ammoniac.

7. Utilisation de microparticules selon la revendication 4 ou 5, pour la préparation d'un agent d'exploration fonctionnelle.

8. Procédé de préparation de microparticules à teneur en gaz pour le diagnostic échographique, dont le matériau de paroi est constitué de polymères à blocs de polyesters d'acides a-, β- ou γ-hydroxycarboxyliques avec des polyéthylène glycols linéaires ou étoilés et le cas échéant de composés liquides cristallins ou de polyesters d'acides a-, β-, ou γ-hydroxycarboxyliques et de composés liquides cristallins, **caractérisé en ce que** le polymère souhaité, le cas échéant, le composé liquide cristallin, et le cas échéant, une substance tensioactive sont dissous dans un solvant organique ou dans des mélanges de solvants, parmi lesquels au moins un solvant est facilement miscible dans l'eau, puis un composé perfluoro gazeux à température du corps ou de l'eau est dispersé dans cette solution et ensuite cette dispersion est dispersée avec un agitateur dans de l'eau contenant une substance tensioactive, tandis que le solvant est éliminé par introduction de gaz et application de vide puis la suspension ainsi obtenue est mélangée avec un cryoprotecteur approprié pharmaccutiquement acceptable puis lyophilisée.

9. Procédé selon la revendication 8, **caractérisé en ce qu'**on utilise comme substance tensioactive des substances du groupe des poloxamères ou poloxamines, polyéthylène glycol alkyléther, polysorbate, ester de saccharose,
gélatine, polyvinylpyrrolidone, polyglycoside d'alcools gras, chaps, chap, chapso, décyl-β-D-glycopyranoside, décyl-β-D-maltopyranoside, dodécyl-β-D-maltopyranoside, oléate de sodium, polyéthylène glycol, alcool polyvinylique ou leurs mélanges.

10. Procédé selon la revendication 8 ou 9, **caractérisé en ce qu'**on utilise comme composé perfluoro le perfluoropentane, le perfluorohexane, le perfluoro-1,3-diméthylcyclohexane, le perfluorocyclohexène, la perfluorodécaline et/ou le perfluoroéther.

11. Procédé de préparation de microparticules à teneur en gaz pour le diagnostic échographique, dont le matériau de paroi est constitué de polymères à blocs de polyesters d'acides a-, β- ou γ-hydroxycarboxyliques avec des polyéthylène glycols linéaires ou étoilés, **caractérisé en ce que** le(s) polymère(s) souhaité(s) et le cas échéant un acide aminé est (sont) dissous dans au moins un solvant organique, cette solution étant injectée par une buse dans une colonne qui est remplie avec un gaz supercritique ou qui est traversée par un courant de celui-ci, tandis que le solvant est repris par le gaz supercritique.

12. Procédé selon la revendication 11, **caractérisé en ce qu'**on utilise comme gaz supercritique de l'oxyde azoté, du dioxyde de carbone, des hydrocarbures halogénés, des hydrocarbures saturés ou insaturés, du dioxyde d'azote et/ou du gaz ammoniac.

13. Procédé selon la revendication 11 ou 12, **caractérisé en ce qu'**on utilise comme acide aminé la L-lysine, la L-phénylalanine, le L-tryptophane ou la D,L-phénylalanine.

14. Procédé selon les revendications 8 à 13, **caractérisé en ce qu'**on utilise comme solvant organique pour le polymère le dichlorométhane, l'acétone, l'acétate d'éthyle, l'acétate de méthyle, la triacétine, le citrate de triéthyle, le lactate d'éthyle, l'acétate d'isopropyle, le formiate de propyle, le formiate de butyle, le formiate d'éthyle et/ou le lactate de méthyle.

15. Procédé de préparation d'agents de contraste pour le diagnostic échographique, **caractérisé en ce que** les microparticules selon les revendications 1 à 6 sont suspendues dans un milieu de suspension pharmaceutiquement compatible.
